## Europäisches Patentamt
## European Patent Office
## Office européen des brevets

(19)

(11) Numéro de publication: **0 045 700**
**B1**

(12) **FASCICULE DE BREVET EUROPEEN**

(45) Date de publication du fascicule du brevet:
**08.08.84**

(51) Int. Cl.³: **A 61 B 5/14**, A 61 B 17/34,
A 61 B 17/42, A 61 M 25/00

(21) Numéro de dépôt: **81401264.7**

(22) Date de dépôt: **05.08.81**

(54) **Dispositif pour la ponction du sang foetal in utero.**

(30) Priorité: **06.08.80 FR 8017416**

(43) Date de publication de la demande:
**10.02.82 Bulletin 82/6**

(45) Mention de la délivrance du brevet:
**08.08.84 Bulletin 84/32**

(84) Etats contractants désignés:
**BE CH DE GB IT LI LU NL SE**

(56) Documents cités:
**US - A - 2 243 285**
**US - A - 3 685 509**
**US - A - 3 810 456**
**US - A - 3 967 625**

(73) Titulaire: **LABORATOIRES BIOTROL S.A., 1, rue du Foin,
F-75140 Paris Cedex 03 (FR)**

(72) Inventeur: **Jeanty, Philippe, 26 Rue du Poirier,
B-7191 Ecaussinnes (BE)**

(74) Mandataire: **Phélip, Bruno et al, c/o Cabinet Harlé &
Phélip 21, rue de la Rochefoucauld, F-75009 Paris (FR)**

BUNDESDRUCKEREI BERLIN

## Description

La présente invention concerne un dispositif pour la ponction du sang foetal in utero, et plus particulièrement un dispositif selon la revendication 1 agencé pour atteindre le cordon ombilical d'un foetus in utero et y prélever une certaine quantité de sang, en vue de l'analyse de celui-ci, notamment pour l'établissement rapide d'un caryotype et/ou la réalisation de toutes autres mesures ou analyses biologiques.

Un dispositif permettant de ponctionner du sang sur un foetus in utero est connu du brevet US-A 3 685 509. Ledit dispositif comprend un premier tube permettant le coulissement d'un trocart, un second tube destiné au frélèvement du sang foetal, et des moyens pour maintenir le dispositif sur la partie à fonctionner.

Avec pour objectif de permettre l'établissement rapide d'un caryotype et/ou la réalisation d'autres mesures ou analyses biologiques, et d'en tirer au plus vite toutes les conséquences importantes qui peuvent être déduite de la lecture des résultats, en particulier quant au bon déroulement ultérieur de la grossesse, aux risques de complications et à la viabilité du foetus, on a maintenant conçu un dispositif permettant d'atteindre et de soisir le cordon ombilical d'un foetus in utero et d'y prélever une certaine quantité de sang, et spécialement agencé à cette fin.

Le dispositif selon l'invention comprend fondamentalement deux tubes parallèles formant canules, un trocart et une tige se terminant par un crochet élastique à une de ses extrémités et qui sont tous deux destinés à coulisser dans le premier des deux tubes et ont une longueur supérieure à celui-ci, ainsi qu'une aiguille creuse destinée à coulisser dans le second des deux tubes et ayant une longueur supérieure à celui-ci, et également des moyens pour positionner ou solidariser temporairement le trocart, la tige munie d'un crochet et/ou l'aiguille creuse à l'extrémité amont du tube respectif dans lequel ils pénètrent.

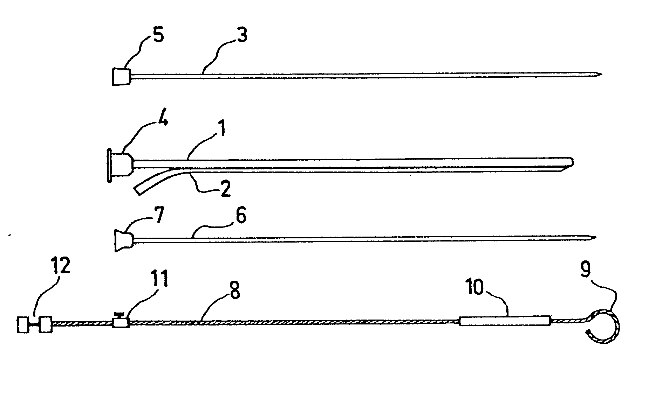

L'invention est décrite ci-après plus en détail en référence à la figure unique annexée, qui représente de manière schématique, et à titre purement illustratif et non limitatif, une forme de mise en oeuvre des différents éléments fondamentaux du dispositif conforme à l'invention, artificiellement séparés et présentés les uns à côté des autres pour en faciliter l'identification.

Les deux tubes 1 et 2 susdits peuvent être constitués de deux tubes cylindriques, de diamètres identiques ou différents, soudés l'un à l'autre selon une génératrice; ils peuvent aussi être ménagés dans un matériau ayant la longueur requise et une section droite, de préférence ovoïde, appropriée pour comporter conjointement les lumières des deux tubes.

Dans l'état actuel de la mise au point du dispositif, et eu égard à l'application considérée, le diamètre extérieur maximal de l'ensemble de deux tubes ainsi constitué ne dépasse pas environ 3 mm. Bien que cela ne soit pas limitatif non plus, la longueur de ces tubes est avantageusement d'environ 15 à 17 cm, tandis que le diamètre intérieur de chacun des tubes est d'environ 1 mm.

L'extrémité aval de cet ensemble des deux tubes, c'est-à-dire l'extrémité qui pénètre dans l'utérus, est de préférence biseautée sur son pourtour de façon à être le plus atraumatique possible.

Le trocart 3, qui est destiné à la perforation de la paroi abdominale, a une structure classique; son extrémité est de préférence taillée en triple biseau. Avantageusement, le tube dans lequel ce trocart coulisse librement porte une embase 4, du côté proximal par rapport à l'utilisateur, et le trocart comporte à son extrémité correspondante un bouchon 5 qui s'engage dans cette embase, en cours d'utilisation. Pour améliorer cette fixation temporaire du trocart et conférer à celui-ci une position invariable par rapport au système de tubes, il est avantageux de munir ledit bouchon d'un ergot et de ménager dans l'embase susdite une fente longitudinale apte à bloquer cet ergot.

L'aiguille creuse de ponction 6, destinée à pénétrer dans le second des deux tubes, doit pouvoir coulisser librement dans celui-ci. Son extrémité proximale, par rapport à l'utilisateur, est de préférence munie d'une embase 7 qui en facilite la préhension et en même temps sert de butée de fin d'introduction dans le tube où cette aiguille creuse est introduite; ce tube peut être muni lui-même d'une embase ou autre système similaire facilitant l'introduction de l'aiguille et, si nécessaire, permettant l'obturation dudit tube. La longueur de cette aiguille creuse doit être telle que, une fois introduite dans le tube jusqu'à sa butée, elle ressorte à l'autre extrémité, dans la pratique d'une longueur suffisante pour permettre la ponction d'un vaisseau du cordon ombilical.

Selon une variante préférée, l'extrémité proximale de ce tube où pénètre l'aiguille creuse de ponction est coudée et s'écarte ainsi du premier tube, cela afin essentiellement de dégager la lumière de ce deuxième tube par rapport aux accessoires que porte l'extrémité proximale du premier tube. Dans ce cas, il est bien entendu que l'aiguille creuse doit être réalisée en un matériau souple, apte à suivre cette courbure du deuxième tube.

La tige 8 se terminant à l'une de ses extrémités par un crochet élastique 9 et qui est destinée à être introduite dans le premier des deux tubes susdits, après que le système a été introduit à proximité du foetus et que le trocart a été enlevé, a pour fonction de saisir le cordon ombilical et de l'amener à proximité immédiate de l'extrémité distale du deuxième tube où on introduit ensuite l'aiguille creuse de ponction qui perfore ainsi la paroi du cordon ombilical et permet des prélèvements du sang qui y circule.

Cette tige doit donc pouvoir être manoeuvrée et déplacée par l'utilisateur d'une part pour faire varier la distance entre le crochet qui la termine et l'extrémité distale du système de tubes, et d'autre part pour faire varier la courbure du crochet. Cela peut être réalisé avec l'aide de tous moyens connus de l'homme de l'art, permettant de s'assurer du déroulement et du succès de la manoeuvre.

On a cependant, à la suite de mises au point successives, pu établir qu'un dispositif préféré selon l'invention comprend une tige creuse constituée d'un enroulement à spires jointives d'un fil souple, notamment un fil métallique, et d'une âme, également de préférence métallique, fixée, après introduction dans la tige creuse, de manière inamovible à l'extrémité distale de celle-ci et dont la partie distale a été traitée pour acquérir au repos une configuration de boucle ou crochet et est douée d'une élasticité suffisante pour pouvoir être étirée en position pratiquement rectiligne et revenir, hors sollicitation, et sa configuration de crochet.

Pour l'application considérée, il convient que ce crochet ait un diamètre intérieur en rapport avec celui du cordon ombilical concerné.

On notera toutefois que la forme et l'ouverture de cette boucle peuvent être quelconques.

Un organe est de préférence prévu pour faciliter à l'utilisateur le redressement en position rectiligne de ce crochet et l'introduction de l'extrémité distale correspondante dans le premier tube. Un tel organe peut être, par exemple, un simple manchon cylindrique 10 coulissant librement sur la tige 9 portant ce crochet et pouvant être ramené vers la partie proximale de celle-ci après introduction dans le premier tube. De plus, le blocage de la tige porteuse du crochet qui arrime le cordon ombilical dans la position nécessaire à sa ponction peut être assuré par un coulisseau 11 se déplaçant librement sur ladite tige et muni d'une vis latérale de fixation permettant de maintenir la tige après que ce coulisseau a été amené en butée sur l'embase du premier tube.

Un dispositif 12 approprié, du type de ceux qui sont connus de l'homme de l'art, assure le déplacement relatif de l'âme susdite par rapport à la tige creuse et, partant, le réglage, à volonté, de l'ouverture du crochet.

Dans la pratique, la manoeuvre du dispositif selon l'invention est subordonnée aux informations reçues au moyen d'un appareillage adéquat, qui peut être un appareil d'échographie ou également un amnioscope.

Pour compléter le dispositif, on relie celui-ci, en cours d'utilisation, à un appareil de recueil de sang.

Les analyses et mesures multiples que permettent les prélèvements de sang foetal ainsi effectués peuvent alors débuter.

## Revendications

1. Dispositif pour la ponction du sang foetal in utéro, comprenant fondamentalement deux tubes parallèles formant canules, (1, 2), un trocart (3) et une tige (8) se terminant par un crochet élastiques à une de ses extrémités et qui sont tous deux destinés à coulisser dans le premier des deux tubes et ont une longueur supérieure à celui-ci, ainsi qu'une aiguille creuse (6) destinée à coulisser dans le second des deux tubes et ayant une longueur supérieure à celui-ci, et également des moyens pour positionner ou solidariser temporairement le trocart, la tige munie d'un crochet et/ou l'aiguille creuse à l'extrémité amont du tube respectif dans lequel ils pénètrent.

2. Dispositif selon la revendication 1, caractérisé en ce que les deux tubes sont constitués de tubes cylindriques, de diamètres identiques ou différents, soudés l'un à l'autre selon une génératrice.

3. Dispositif selon l'une des revendications 1 ou 2, caractérisé en ce que la tige (8) est essentiellement composée d'une tige creuse constituée d'un enroulement à spires jointives d'un fil souple et d'une âme, fixée, après introduction dans la tige creuse, de manière inamovible à l'extrémité distale de celle-ci et dont la partie distale a été traitée pour acquérir au repos une configuration de boucle ou crochet et est douée d'une élasticité suffisante pour pouvoir être étirée en position pratiquement rectiligne et revenir, hors sollicitation, à sa configuration de crochet.

4. Dispositif selon l'une quelconque des revendications 1 à 3, caractérisé en ce qu'il comprend un manchon cylindrique coulissant librement sur la tige portant le crochet.

## Patentansprüche

1. Vorrichtung für die fötale Blutentnahme in utero, umfassend grundsätzlich zwei parallele, Kanülen bildende Rohr (1, 2), einen Trokar (3) und eine Stange (8), die an einem ihrer Enden in einen elastischen Haken ausläuft, welche beiden für die Gleitbewegung in dem ersten der beiden Rohre ausgebildet sind und eine dessen Länge übersteigende Länge besitzen, sowie eine Hohlnadel (6) zur Gleitbewegung im zweiten Rohr ausgebildet mit einer dessen Länge übersteigenden Länge sowie mit Mitteln zum Positionieren oder zeitweiligen Festlegen des Trokars, der mit einem Haken versehenen Stange und/oder der Hohlnadel an demjenigen Vorderende des Rohres, in das sie jeweils eindringen.

2. Vorrichtung nach Anspruch 1, dadurch gekennzeichnet, daß die beiden Rohre von zwei zylindrischen Rohren gleichen oder unterschiedlichen Durchmessers gebildet sind, die miteinander längs einer Mantellinie verlötet oder verschweißt sind.

3. Vorrichtung nach einem der Ansprüche 1 oder 2, dadurch gekennzeichnet, daß die Stange (8) im wesentlichen von einer Hohlstange gebildet ist, bestehend aus einem Wickel aneinanderliegender Spiralen eines weichen Drahtes sowie aus einer Seele, die nach Einführen in die Hohlstange unbeweglich am distalen Ende derselben befestigt ist und deren distales Ende derart vorbehandelt ist, daß es im Ruhezustand eine schlaufen- oder hakenförmige Konfiguration annimmt, und die hinreichend elastisch ist, um in eine praktisch gerade Position gestreckt zu werden und bei fehlender Betätigung in die Hakenkonfiguration zurückzukehren.

4. Vorrichtung nach einem der Ansprüche 1 bis 3, dadurch gekennzeichnet, daß sie eine auf der den Haken tragenden Stange frei verschiebliche zylindrische Hülse umfaßt.

**Claims**

1. A device to puncture foetal blood in utero comprising basically two parallel tubes forming cannula (1, 2), a trocar (3) and a stem (8) ending at one end in a resilient hook, both of them being designed to slide within the first tube and being longer than said tube, together with a hollow needle (6) designed to slide within the second tube and being longer than said tube, together with means for positioning or removably interlocking the trocar, the hook-bearing stem, and/ or the hollow needle to the proximal ending of the respective tube wherein they slide.

2. Device according to claim 1, characterized in that the two tubes are cylindrical tubes, of identical or different diameters, fixed along a generatrix.

3. Device according to any of claims 1 or 2, characterized in that the stem (8) essentially consists of a hollow stem resulting from winding of a flexible cord made of contiguous turns, and of a core unremovably fixed, after its insertion into the hollow stem, to its distal ending of said stem, distal portion of the core having been treated to assume at rest a loop or hook configuration and having sufficient resiliency to be able of being stretched to a linear form and of resuming, upon stress removal, its hook configuration.

4. Device according to any of claims 1 to 3 characterized in that it comprises a cylindrical sleeve free to slide on hook stem.